(19) Europäisches Patentamt / European Patent Office / Office européen des brevets

(11) **EP 4 670 840 A1**

(12) **EUROPEAN PATENT APPLICATION**
published in accordance with Art. 153(4) EPC

(43) Date of publication:
**31.12.2025 Bulletin 2026/01**

(21) Application number: **24760066.1**

(22) Date of filing: **31.01.2024**

(51) International Patent Classification (IPC):
*B01J 20/26* [(2006.01)]   *A61L 9/014* [(2006.01)]
*C08K 5/09* [(2006.01)]   *C08K 7/22* [(2006.01)]
*C08L 101/14* [(2006.01)]

(52) Cooperative Patent Classification (CPC):
A61L 9/014; B01J 20/26; C08K 5/09; C08K 7/22;
C08L 101/14

(86) International application number:
**PCT/JP2024/003049**

(87) International publication number:
**WO 2024/176759 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.02.2023 JP 2023026374**

(71) Applicant: **Sumitomo Seika Chemicals Co., Ltd. Kako-gun, Hyogo 675-0145 (JP)**

(72) Inventor: **MORISHIMA, Shota Himeji-shi, Hyogo 672-8076 (JP)**

(74) Representative: **Mewburn Ellis LLP Aurora Building Counterslip Bristol BS1 6BX (GB)**

(54) **WATER-ABSORBING RESIN COMPOSITION**

(57) There is provided a water-absorbent resin composition having an excellent deodorizing effect. A water-absorbent resin composition comprising an organic acid, a porous deodorant, and water-absorbent polymer particles, wherein a ratio (X/Y) of an amount X (parts by mass) of the organic acid to an amount Y (parts by mass) of the porous deodorant is 1.0 or more, and wherein a sum (x + y) of a content x (% by mass) of the organic acid and a content y (% by mass) of the porous deodorant in the total water-absorbent resin composition is 0.5% by mass or more.

[FIG. 1]

EP 4 670 840 A1

**Description**

Technical Field

The present invention relates to a water-absorbent resin composition, and more particularly to a water-absorbent resin composition that constitutes an absorbent material suitably used for hygienic materials, such as disposable diapers, sanitary napkins, and incontinence pads.

Background Art

In recent years, water-absorbent resins have been widely used in the field of hygienic materials, such as disposable diapers, sanitary napkins, and incontinence pads.

As such water-absorbent resins, cross-linked polymers having structural units derived from acrylic acid and neutralized salts thereof are known and have an excellent water-absorption capacity. Since acrylic acid used as a raw material of the cross-linked polymers is readily industrially available, the cross-linked polymers have been proposed as preferable water-absorbent resins because they have many advantages, for example, they can be produced at low cost with uniform quality, and additionally are resistant to decomposition or degradation.

An absorbent article, such as a disposable diaper, a sanitary napkin, or an incontinence pad, is composed of an absorbent material that absorbs and retains a body liquid, such as urine or menses excreted from the body, the absorbent material being positioned mainly in a central portion, a liquid-permeable front sheet (top sheet) positioned on the side of the absorbent article that is brought into contact with the body, and a liquid-impermeable rear sheet (back sheet) positioned opposite to the side that is brought into contact with the body. The absorbent material is typically composed of hydrophilic fibers, such as pulp, and a water-absorbent resin.

When this absorbent material is used in, for example, a hygienic material, an unpleasant odor such as ammonia may be produced from the absorbent material that has absorbed a body fluid, particularly urine, blood, sweat, or the like.

Citation List

Patent Literature

Patent Literature 1: JP-A-2001-323155

Summary of Invention

Technical Problem

When urine is absorbed by the absorbent material, urea in the urine is decomposed by the action of urease of urease-producing bacteria to generate ammonia.

The present inventor has attempted to prevent the generation of ammonia, by lowering the pH of urine using an organic acid to inactivate the urease, thereby inhibiting the urease-induced urea decomposition reaction. However, as a result of research, the present inventor has found that the pH buffering action by the water-absorbent polymer particles in the absorbent material causes the pH of urine lowered by the organic acid to shift to the neutral side, which weakens the urease-inactivating action by the organic acid, so that the deodorizing effect is not fully exhibited.

It is a main object of the present invention to provide a water-absorbent resin composition that has an excellent deodorizing effect.

Solution to Problem

The present inventor has conducted extensive research to solve the aforementioned problem. As a result, the present inventor has found that when an organic acid and a porous deodorant are used in combination in a water-absorbent resin composition containing water-absorbent polymer particles, and furthermore, a ratio (X/Y) of an amount X (parts by mass) of the organic acid to an amount Y (parts by mass) of the porous deodorant is set to equal to or more than a predetermined ratio, and also a sum (x + y) of a content x (% by mass) of the organic acid and a content y (% by mass) of the porous deodorant in the total water-absorbent resin composition is set to equal to or more than a predetermined proportion, such a water-absorbent resin composition exhibits a high deodorizing function against ammonia. The present invention has been accomplished as a result of further research based on these findings.

In summary, the present invention provides aspects of the invention comprising the following features:

Item 1. A water-absorbent resin composition comprising an organic acid, a porous deodorant, and water-absorbent polymer particles having a structural unit derived from a neutralized salt of an ethylenically unsaturated monomer,

wherein a ratio (X/Y) of an amount X (parts by mass) of the organic acid to an amount Y (parts by mass) of the porous deodorant is 1.0 or more, and
wherein a sum (x + y) of a content x (% by mass) of the organic acid and a content y (% by mass) of the porous deodorant in the total water-absorbent resin composition is 0.50% by mass or more.

Item 2. The water-absorbent resin composition according to Item 1, wherein the ratio (X/Y) of the amount X (parts by mass) to the amount Y (parts by mass) is 12.0 or less.

Item 3. The water-absorbent resin composition according to Item 1 or 2, wherein the sum (x + y) of the content x (% by mass) of the organic acid and the content y (% by mass) of the porous deodorant is 1.50% by mass or less.

Item 4. An absorbent article comprising an organic acid, a porous deodorant, and water-absorbent polymer particles,

wherein a ratio (X/Y) of an amount X (parts by mass) of the organic acid to an amount Y (parts by mass) of the porous deodorant is 1.0 or more, and
wherein a sum (x + y) of a content x (% by mass) of the organic acid and a content y (% by mass) of the porous deodorant based on a total mass of the organic acid, the porous deodorant, and the water-absorbent polymer particles is 0.5% by mass or more.

Advantageous Effects of Invention

According to the present invention, it is possible to provide a water-absorbent resin composition that has an excellent deodorizing effect.

Brief Description of Drawings

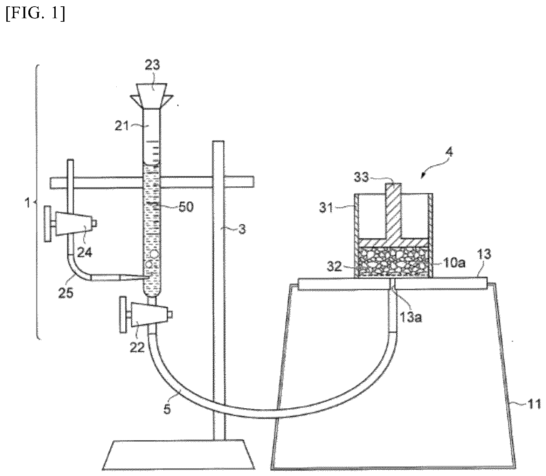

Fig. 1 is a schematic diagram of a measurement apparatus for physiological saline absorption amount under a load of 4.14 kPa.

Description of Embodiments

As used herein, the term "comprising" includes "consisting essentially of" and "consisting of". As used herein, the term "(meth)acrylic" refers to "acrylic or methacrylic", and the term "(meth)acrylate" refers to "acrylate or methacrylate". As used herein, the term "water-soluble" refers to having a water solubility of 5% by mass or more at 25°C.

As used herein, values connected with "to" refer to the numerical range including the values before and after "to" as the lower and upper limits. When a plurality of lower limits and a plurality of upper limits are mentioned separately, any lower limit and any upper limit may be selected and connected with "to".

1. Water-Absorbent Resin Composition

The water-absorbent resin composition of the present invention is a water-absorbent resin composition comprising an organic acid, a porous deodorant, and water-absorbent polymer particles, wherein a ratio (X/Y) of an amount X (parts by mass) of the organic acid to an amount Y (parts by mass) of the porous deodorant is 1.0 or more, and wherein a sum (x + y) of a content x (% by mass) of the organic acid and a content y (% by mass) of the porous deodorant in the total water-absorbent resin composition is 0.5% by mass or more. The water-absorbent resin composition of the present invention having these features exhibits an excellent deodorizing effect. The water-absorbent resin composition of the present invention will be hereinafter described in detail.

As described above, the present inventor has attempted to prevent the generation of ammonia, by lowering the pH of urine using an organic acid to inactivate the urease, thereby inhibiting the urease-induced urea decomposition reaction. However, as a result of research, the present inventor has found that the pH buffering action by the water-absorbent polymer particles in the absorbent material causes the pH of urine lowered by the organic acid to shift to the neutral side, which weakens the urease-inactivating action by the organic acid, so that the deodorizing effect is not fully exhibited. As a result of further research, the present inventor has found that when an organic acid and a porous deodorant are used in combination in a water-absorbent resin composition containing water-absorbent polymer particles, and then the ratio (X/Y) of the amount X (parts by mass) of the organic acid to the amount Y (parts by mass) of the porous deodorant is set to 1.0 or more, and also the sum (x + y) of the content x (% by mass) of the organic acid and the content y (% by mass) of the porous deodorant in the total water-absorbent resin composition is set to 0.5% by mass or more, the organic acid and the porous deodorant work synergistically, so that the water-absorbent resin composition exhibits a high deodorizing function against ammonia. This can be attributed to the fact that the porous deodorant prevents the pH buffering action by the water-absorbent polymer particles (more specifically, the pH buffering action by the moiety derived from the neutralized salt of the water-soluble ethylenically unsaturated monomer contained as a structural unit of the water-absorbent polymer particles), so that the urease-inactivating action by the organic acid is satisfactorily exhibited, and the water-absorbent resin composition exhibits a high deodorizing function against ammonia or the like.

In the water-absorbent resin composition of the present invention, at least a portion of the organic acid (for example, 20% by mass to 100 % by mass, 50% by mass to 100% by mass, 80% by mass to 100% by mass, 90% by mass to 100% by mass, 95% by mass to 100% by mass, or 100% by mass of the total organic acid) may be disposed on the surface of the water-absorbent polymer particles, or at least a portion of the organic acid may penetrate into the water-absorbent polymer particles. In addition, in the water-absorbent resin composition of the present invention, at least a portion of the porous deodorant (for example, 20% by mass to 100% by mass, 50% by mass to 100% by mass, 80% by mass to 100% by mass, 90% by mass to 100% by mass, 95% by mass to 100% by mass, or 100% by mass of the total porous deodorant) may be disposed on the surface of the water-absorbent polymer particles. In the water-absorbent resin composition of the present invention, at least a portion of the organic acid and at least a portion of the porous deodorant may be disposed on the surface of the water-absorbent polymer particles.

(Organic Acid)

The organic acid is a component that lowers the pH of urine absorbed by the absorbent material.

The pH of a 10% by mass aqueous solution of the organic acid as measured by the below-described method may be, for example, 1.0 to 6.0, 1.0 to 4.0, 1.0 to 3.0, 1.0 to 2.0, 1.5 to 6.0, 1.5 to 4.0, 1.5 to 3.0, or 1.5 to 2.0.

The organic acid may contain at least one selected from the group consisting of, for example, tartaric acid, citric acid, malic acid, maleic acid, fumaric acid, succinic acid, acetic acid, and propionic acid, and may contain at least one selected from the group consisting of tartaric acid, citric acid, malic acid, and maleic acid. The organic acid preferably contains at least tartaric acid, which is presumed by the present inventor to be unlikely to be adsorbed by the porous deodorant (i.e., to be likely to lower the pH of urine). In this case, the proportion of tartaric acid may be 70% by mass to 100% by mass, 80% by mass to 100% by mass, 90% by mass to 100% by mass, or 95% by mass to 100% by mass in the organic acid. Tartaric acid is preferably the L-form of tartaric acid, which is considered to be unlikely to be adsorbed by the porous deodorant. The organic acid contained in the water-absorbent resin composition may be one organic acid or two or more organic acids.

In view of more satisfactorily achieving the effect of the present invention, when the organic acid is in particulate form, the organic acid may have a median particle size (D50 (median size), volume-based) of 20 $\mu$m to 600 $\mu$m, 20 $\mu$m to 500 $\mu$m, 20 $\mu$m to 400 $\mu$m, 20 $\mu$m to 300 $\mu$m, 50 $\mu$m to 600 $\mu$m, 50 $\mu$m to 500 $\mu$m, 50 $\mu$m to 400 $\mu$m, 50 $\mu$m to 300 $\mu$m, 100 $\mu$m to 600 $\mu$m, 100 $\mu$m to 500 $\mu$m, 100 $\mu$m to 400 $\mu$m, 100 $\mu$m to 300 $\mu$m, 150 $\mu$m to 600 $\mu$m, 150 $\mu$m to 500 $\mu$m, 150 $\mu$m to 400 $\mu$m, or 150 $\mu$m to 300 $\mu$m. The median particle size (D50 (median size), volume-based) of the organic acid can be measured using a laser diffraction particle size distribution analyzer.

The sum (x + y) of the content x (% by mass) of the organic acid and the content y (% by mass) of the porous deodorant in the total water-absorbent resin composition of the present invention is 0.50% by mass or more. In view of more satisfactorily achieving the effect of the present invention, the sum (x + y) may be 0.50% by mass to 2.00% by mass, 0.50% by mass to 1.50% by mass, 0.50% by mass to 1.40% by mass, 0.50% by mass to 1.30% by mass, 0.50% by mass to 1.20% by mass, 0.50% by mass to 1.00% by mass, 0.53% by mass to 2.00% by mass, 0.53% by mass to 1.50% by mass, 0.53% by mass to 1.40% by mass, 0.53% by mass to 1.30% by mass, 0.53% by mass to 1.20% by mass, 0.53% by mass to 1.00% by mass, 0.56% by mass to 2.00% by mass, 0.56% by mass to 1.50% by mass, 0.56% by mass to 1.40% by mass, 0.56% by mass to 1.30% by mass, 0.56% by mass to 1.20% by mass, 0.56% by mass to 1.00% by mass, 0.59% by mass to 2.00% by mass, 0.59% by mass to 1.50% by mass, 0.59% by mass to 1.40% by mass, 0.59% by mass to 1.30% by mass, 0.59% by mass to 1.20% by mass, or 0.59% by mass to 1.00% by mass. When the organic acid is in particulate form, a larger sum (x + y) of the content x (% by mass) of the organic acid and the content y (% by mass) of the porous deodorant is more likely to cause the organic acid and the porous deodorant to contact and rub against each other, so that fine powder is more likely to be generated (in other words, the degree of dusting increases), resulting in reduced handleability of the water-absorbent resin composition. In view of the degree of dusting, the sum (x + y) is preferably 1.50% by mass or less.

In view of more satisfactorily achieving the effect of the present invention, the content x (% by mass) of the organic acid in the total water-absorbent resin composition of the present invention may be 0.30% by mass to 1.20% by mass, 0.30% by mass to 1.00% by mass, 0.30% by mass to 0.80% by mass, 0.40% by mass to 1.20% by mass, 0.40% by mass to 1.00% by mass, 0.40% by mass to 0.80% by mass, 0.45% by mass to 1.20% by mass, 0.45% by mass to 1.00% by mass, or 0.45% by mass to 0.80% by mass. A higher content x (% by mass) of the organic acid tends to lower the water-absorption rate of the water-absorbent resin composition. In consideration of this, the upper limit of the content x (% by mass) is preferably 1.00% by mass.

In the water-absorbent resin composition of the present invention, the ratio (X/Y) of the amount X (parts by mass) of the organic acid to the amount Y (parts by mass) of the porous deodorant is 1.0 or more. In view of more satisfactorily achieving the effect of the present invention, the ratio (X/Y) may be 1.0 to 15.0, 1.0 to 12.0, 1.0 to 10.0, 1.0 to 8.0, 1.0 to 6.0, 1.5 to 15.0, 1.5 to 12.0, 1.5 to 10.0, 1.5 to 8.0, 1.5 to 6.0, 2.0 to 15.0, 2.0 to 12.0, 2.0 to 10.0, 2.0 to 8.0, 2.0 to 6.0, 2.5 to 15.0, 2.5 to 12.0, 2.5 to 10.0, 2.5 to 8.0, 2.5 to 6.0, 3.0 to 15.0, 3.0 to 12.0, 3.0 to 10.0, 3.0 to 8.0, or 3.0 to 6.0. The upper limit of the ratio (X/Y) is preferably 12.0, in consideration of the effect of the organic acid on the water-absorption rate of the water-absorbent resin composition.

As described above, in the water-absorbent resin composition of the present invention, when the organic acid is in particulate form, the organic acid may be disposed on the surface of the water-absorbent polymer particles (i.e., the organic acid may be present on the surface of the water-absorbent polymer particles). The organic acid can be disposed on the surface of the water-absorbent polymer particles by, for example, mixing the water-absorbent polymer particles and the particulate organic acid in a solid phase, so that the organic acid is adhered to the surface of the water-absorbent polymer particles.

(Porous Deodorant)

The porous deodorant is a deodorant that is porous.

In view of more satisfactorily achieving the effect of the present invention, the porous deodorant may contain at least one selected from the group consisting of zeolite, activated carbons, silicon dioxide, silicates, titania, alumina, aluminum hydroxide, and magnesium hydroxide, or may contain at least one selected from the group consisting of zeolite, activated carbons, and silicon dioxide. In particular, the porous deodorant preferably contains at least an activated carbon, which is presumed to be unlikely to adsorb the organic acid, and the proportion (% by mass) of the activated carbon is, for example, 80% by mass to 100% by mass, 90% by mass to 100% by mass, or 95% by mass to 100% by mass.

The porous deodorant may have a median particle size of 1 $\mu$m to 100 $\mu$m, 1 $\mu$m to 80 $\mu$m, 1 $\mu$m to 60 $\mu$m, 10 $\mu$m to 100 $\mu$m, 10 $\mu$m to 80 $\mu$m, 10 $\mu$m to 60 $\mu$m, 15 $\mu$m to 100 $\mu$m, 15 $\mu$m to 80 $\mu$m, 15 $\mu$m to 60 $\mu$m, 20 $\mu$m to 100 $\mu$m, 20 $\mu$m to 80 $\mu$m, or 20 $\mu$m to 60 $\mu$m.

The median particle size (D50 (median size), volume-based) of the porous deodorant can be measured using a laser diffraction particle size distribution analyzer; specifically, it is the value as measured by the method described in the Examples section.

The porous deodorant has a shape such as, for example, a crushed shape or a cylindrical shape, preferably a crushed shape.

The porous deodorant may have a BET specific surface area of 100 $m^2$/g to 3000 $m^2$/g, 100 $m^2$/g to 2500 $m^2$/g, 100 $m^2$/g to 2000 $m^2$/g, 100 $m^2$/g to 1500 $m^2$/g, 500 $m^2$/g to 3000 $m^2$/g, 500 $m^2$/g to 2500 $m^2$/g, 500 $m^2$/g to 2000 $m^2$/g, 500 $m^2$/g to 1500 $m^2$/g, 1000 $m^2$/g to 3000 $m^2$/g, 1000 $m^2$/g to 2500 $m^2$/g, 1000 $m^2$/g to 2000 $m^2$/g, or 1000 $m^2$/g to 1500 $m^2$/g. If the BET specific surface area of the porous deodorant is too large, each pore may be so fine that the strength of the porous deodorant decreases and the above-described degree of dusting increases. Therefore, the upper limit of the BET specific surface area is preferably 2000 $m^2$/g.

The BET specific surface area of the porous deodorant can be measured using a specific surface area analyzer; specifically, it is the value as measured by the method described in the Examples section.

In view of more satisfactorily achieving the effect of the present invention, the activated carbon serving as the porous deodorant is preferably an activated carbon having a polar functional group (hydrophilic functional group) on the surface (i.e., a hydrophilic activated carbon). Examples of polar functional groups include hydroxy, carboxy, and phenol groups. Activated carbons having polar functional groups on the surface are commercially available as, for example, activated carbons for liquid phase applications and activated carbons for water treatment.

The activated carbon may be produced from, for example, coconut shells, infusibilized or carbonized organic materials, and infusible resins such as phenol resins. Examples of the organic materials include polyacrylonitrile, pitch, polyvinyl alcohol, and cellulose. Among these, the activated carbon is preferably produced from wood (sawdust), coconut shells, or pitch (such as coal pitch).

In view of more satisfactorily achieving the effect of the present invention, the content y (% by mass) of the porous deodorant in the water-absorbent resin composition of the present invention may be 0.05% by mass to 0.50% by mass, 0.05% by mass to 0.40% by mass, 0.05% by mass to 0.35% by mass, 0.05% by mass to 0.30% by mass, 0.07% by mass to 0.50% by mass, 0.07% by mass to 0.40% by mass, 0.07% by mass to 0.35% by mass, 0.07% by mass to 0.30% by mass, 0.08% by mass to 0.50% by mass, 0.08% by mass to 0.40% by mass, 0.08% by mass to 0.35% by mass, 0.08% by mass to 0.30% by mass, 0.10% by mass to 0.50% by mass, 0.10% by mass to 0.40% by mass, 0.10% by mass to 0.35% by mass, 0.10% by mass to 0.30% by mass, 0.15% by mass to 0.50% by mass, 0.15% by mass to 0.40% by mass, 0.15% by mass to 0.35% by mass, 0.15% by mass to 0.30% by mass, 0.20% by mass to 0.50% by mass, 0.20% by mass to 0.40% by mass, 0.20% by mass to 0.35% by mass, 0.20% by mass to 0.30% by mass, 0.25% by mass to 0.50% by mass, 0.25% by mass to 0.40% by mass, 0.25% by mass to 0.35% by mass, or 0.25% by mass to 0.30% by mass.

The porous deodorant may have a loss on drying of, for example, 0.1% to 15.0%, 0.1% to 10.0%, 0.1% to 5.0%, 0.5% to 15.0%, 0.5% to 10.0%, 0.5% to 5.0%, 1.0% to 15.0%, 1.0% to 10.0%, or 1.0% to 5.0%.

As used herein, the loss on drying of the porous deodorant is the value as measured according to JIS K 1474: 2014.

The porous deodorant may have a pH of, for example, 2.0 to 12.0, 2.0 to 11.0, 2.0 to 8.0, 2.0 to 6.0, 2.0 to 5.0, 3.0 to 12.0, 3.0 to 11.0, 3.0 to 8.0, 3.0 to 6.0, 3.0 to 5.0, 4.0 to 12.0, 4.0 to 11.0, 4.0 to 8.0, 4.0 to 6.0, or 4.0 to 5.0. When the porous deodorant is acidic, the porous deodorant and the organic acid can cooperate to further facilitate the inactivation of urease to more satisfactorily achieve the effect of the present invention. In view of this, the upper limit of the pH of the porous deodorant may be 6.0 or 5.0.

As used herein, the pH of the porous deodorant is the value as measured according to JIS K 1474: 2014.

As described above, in the water-absorbent resin composition of the present invention, preferably, the porous deodorant is disposed on the surface of the water-absorbent polymer particles (i.e., the porous deodorant is present on the surface of the water-absorbent polymer particles). The porous deodorant can be disposed on the surface of the water-absorbent polymer particles by, for example, mixing the water-absorbent polymer particles and the porous deodorant in a solid phase, so that the porous deodorant is adhered to the surface of the water-absorbent polymer particles.

Next, the water-absorbent polymer particles (water-absorbent resin particles) contained in the water-absorbent resin composition of the present invention will be described in detail.

(Water-Absorbent Polymer Particles)

The water-absorbent polymer particles contained in the water-absorbent resin composition of the present invention are formed of a crosslinked product of a polymer of a water-soluble ethylenically unsaturated monomer, that is, a crosslinked polymer having a structural unit derived from a water-soluble ethylenically unsaturated monomer.

The water-absorbent polymer particles may have a water-absorption rate as measured by a Vortex method of, for example, 1 second to 80 seconds, 1 second to 60 seconds, 1 second to 40 seconds, 10 seconds to 80 seconds, 10 seconds to 60 seconds, 10 seconds to 40 seconds, 20 seconds to 80 seconds, 20 seconds to 60 seconds, or 20 seconds to 40 seconds.

The water-absorption rate of the water-absorbent polymer particles as measured by the Vortex method is the value as measured by the method described in the Examples section.

The water-absorbent polymer particles may have a physiological saline retention amount of, for example, 20 g/g to 60 g/g, 20 g/g to 55 g/g, 20 g/g to 50 g/g, 25 g/g to 60 g/g, 25 g/g to 55 g/g, 25 g/g to 50 g/g, 30 g/g to 60 g/g, 30 g/g to 55 g/g, or 30 g/g to 50 g/g.

The water-absorbent polymer particles may have a physiological saline absorption amount under a load of 4.14 kPa of, for example, 10 mL/g to 40 mL/g, 10 mL/g to 35 mL/g, 10 mL/g to 30 mL/g, 13 mL/g to 40 mL/g, 13 mL/g to 35 mL/g, 13 mL/g to 30 mL/g, 15 mL/g to 40 mL/g, 15 mL/g to 35 mL/g, or 15 mL/g to 30 mL/g.

The physiological saline retention amount and the physiological saline absorption amount under a load of 4.14 kPa of the water-absorbent polymer particles are each the value as measured by the method described in the Examples section.

The water-absorbent polymer particles may have a median particle size of, for example, 150 $\mu$m to 850 $\mu$m, 150 $\mu$m to 600 $\mu$m, 150 $\mu$m to 550 $\mu$m, 150 $\mu$m to 500 $\mu$m, 150 $\mu$m to 450 $\mu$m, 150 $\mu$m to 400 $\mu$m, 200 $\mu$m to 850 $\mu$m, 200 $\mu$m to 600 $\mu$m, 200 $\mu$m to 550 $\mu$m, 200 $\mu$m to 500 $\mu$m, 200 $\mu$m to 450 m, 200 $\mu$m to 400 $\mu$m, 240 $\mu$m to 850 $\mu$m, 240 $\mu$m to 600 $\mu$m, 240 $\mu$m to 550 $\mu$m, 240 $\mu$m to 500 $\mu$m, 240 $\mu$m to 450 $\mu$m, 240 $\mu$m to 400 $\mu$m, 260 $\mu$m to 850 $\mu$m, 260 $\mu$m to 600 $\mu$m, 260 $\mu$m to 550 $\mu$m, 260 $\mu$m to 500 $\mu$m, 260 $\mu$m to 450 $\mu$m, 260 $\mu$m to 400 $\mu$m, 280 $\mu$m to 850 $\mu$m, 280 $\mu$m to 600 $\mu$m, 280 $\mu$m to 550 $\mu$m, 280 $\mu$m to 500 $\mu$m, 280 $\mu$m to 450 $\mu$m, 280 $\mu$m to 400 $\mu$m, 300 $\mu$m to 850 $\mu$m, 300 $\mu$m to 600 $\mu$m, 300 $\mu$m to 550 $\mu$m, 300 $\mu$m to 500 $\mu$m, 300 $\mu$m to 450 $\mu$m, or 300 $\mu$m to 400 $\mu$m.

The water-absorbent polymer particles may be in the form of particles each composed of a single particle, or in the form of particles (secondary particles) formed by agglomeration of fine particles (primary particles). Examples of the shape of the primary particles include a substantially spherical shape, a crushed indefinite shape, and a flat shape. When the water-absorbent polymer particles are primary particles produced by reversed phase suspension polymerization, examples of the shape include a substantially spherical single-particle shape with a smooth surface shape, such as a spherical shape or an elliptical sphere shape.

The median particle size of the water-absorbent polymer particles can be measured using JIS standard sieves; specifically, it is the value as measured by the method described in the Examples section.

The water-absorbent polymer particles have a structural unit derived from a neutralized salt of the water-soluble ethylenically unsaturated monomer. A representative polymerization method, such as aqueous polymerization, emulsion polymerization, or reversed phase suspension polymerization, is used for polymerizing the water-soluble ethylenically unsaturated monomer. In aqueous polymerization, polymerization is performed by heating, optionally with stirring, an aqueous solution of the water-soluble ethylenically unsaturated monomer. In reversed phase suspension polymerization, polymerization is performed by heating the water-soluble ethylenically unsaturated monomer with stirring in a hydrocarbon dispersion medium.

One exemplary method for producing the water-absorbent polymer particles will be hereinafter described.

Specific examples of methods for producing the water-absorbent polymer particles include a method for producing the water-absorbent polymer particles by performing reversed phase suspension polymerization of the water-soluble ethylenically unsaturated monomer in a hydrocarbon dispersion medium, the method including the steps of performing the polymerization in the presence of a radical polymerization initiator; and surface-crosslinking a hydrous gel obtained by the polymerization, in the presence of a surface-crosslinking agent. In the method for producing the water-absorbent polymer particles of the present invention, an internal-crosslinking agent may be optionally added to the water-soluble ethylenically unsaturated monomer to obtain a hydrous gel having an internal-crosslinked structure.

<Polymerization Step>

[Water-Soluble Ethylenically Unsaturated Monomer]

Examples of the water-soluble ethylenically unsaturated monomer include (meth)acrylic acid ("acryl" and "methacryl" are herein collectively referred to as "(meth)acryl"; the same applies below) and salts thereof; 2-(meth)acrylamido-2-methylpropanesulfonic acid and salts thereof; nonionic monomers, such as (meth)acrylamide, N,N-dimethyl(meth)acrylamide, 2-hydroxyethyl(meth)acrylate, N-methylol(meth)acrylamide, and polyethylene glycol mono(meth)acrylate; and amino group-containing unsaturated monomers, such as N,N-diethylaminoethyl (meth)acrylate, N,N-diethylaminopropyl (meth)acrylate, and diethylaminopropyl (meth)acrylamide, as well as quaternary compounds thereof. Preferred among these water-soluble ethylenically unsaturated monomers are (meth)acrylic acid and salts thereof, (meth)acrylamide, and N,N-dimethyl(meth)acrylamide, and more preferred are (meth)acrylic acid and salts thereof, because they are readily industrially available, for example. These water-soluble ethylenically unsaturated monomers may be used alone or in combinations of two or more.

Among these water-soluble ethylenically unsaturated monomers, acrylic acid and salts thereof are widely used as raw materials of water-absorbent polymer particles. Copolymers of acrylic acid and/or salts thereof with other water-soluble ethylenically unsaturated monomers as mentioned above may also be used. In this case, acrylic acid and/or a salt thereof as a main water-soluble ethylenically unsaturated monomer is preferably used in an amount of 70 to 100 mol% based on the total amount of water-soluble ethylenically unsaturated monomers.

The water-soluble ethylenically unsaturated monomer may be dispersed as an aqueous solution in a hydrocarbon dispersion medium and then subjected to reversed phase suspension polymerization. When the water-soluble ethylenically unsaturated monomer is in the form of an aqueous solution, the dispersion efficiency in the hydrocarbon dispersion medium can be increased. The concentration of the water-soluble ethylenically unsaturated monomer in the aqueous solution is preferably in the range of 20% by mass to not more than the saturation concentration. The concentration of the water-soluble ethylenically unsaturated monomer is more preferably 55% by mass or less, still more preferably 50% by mass or less, and even more preferably 45% by mass or less. On the other hand, the concentration of the water-soluble ethylenically unsaturated monomer is more preferably 25% by mass or more, still more preferably 28% by mass or more, and even more preferably 30% by mass or more.

When the water-soluble ethylenically unsaturated monomer has an acid group such as (meth)acrylic acid or 2-(meth)acrylamido-2-methylpropanesulfonic acid, the acid group may be optionally neutralized with an alkaline neutralizing agent, before the water-soluble ethylenically unsaturated monomer is used. Examples of such alkaline neutralizing agents include alkali metal salts, such as sodium hydroxide, sodium carbonate, sodium hydrogen carbonate, potassium hydroxide, and potassium carbonate; and ammonia. These alkaline neutralizing agents may be used in the form of an aqueous solution to facilitate the neutralization operation. The above-mentioned alkaline neutralizing agents may be used alone or in combinations of two or more.

The degree of neutralization of the water-soluble ethylenically unsaturated monomer with an alkaline neutralizing agent, calculated as the degree of neutralization of all acid groups in the water-soluble ethylenically unsaturated monomer, is preferably 10 to 100 mol%, more preferably 30 to 90 mol%, still more preferably 40 to 85 mol%, and even more preferably 50 to 80 mol%.

[Radical Polymerization Initiator]

Examples of the radical polymerization initiator to be added in the polymerization step include persulfates, such as potassium persulfate, ammonium persulfate, and sodium persulfate; peroxides, such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds, such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis {2-[1-(2-hydroxyethyl)-2-imidazolin-2-yl]propane} dihydrochloride, 2,2'-azobis {2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Preferred among these radical polymerization initiators are potassium persulfate, ammonium persulfate, sodium persulfate, and 2,2'-azobis(2-amidinopropane) dihydrochloride, because they are readily available and easy to handle. These radical polymerization initiators may be used alone or in combinations of two or more. The above-mentioned radical polymerization initiators may also be used in combination with a reducing agent, such as sodium sulfite, sodium hydrogensulfite, ferrous sulfate, or L-ascorbic acid, and used as a redox polymerization initiator.

The amount of the radical polymerization initiator to be used is, for example, 0.00005 to 0.01 mol per mole of the water-soluble ethylenically unsaturated monomer. When the radical polymerization initiator is used in the above-defined range of amounts, the occurrence of an abrupt polymerization reaction can be avoided, and the polymerization reaction can be completed in an appropriate period of time.

[Internal-Crosslinking Agent]

Examples of the internal-crosslinking agent include those that can crosslink the polymer of the water-soluble ethylenically unsaturated monomer to be used, for example: unsaturated polyesters obtained by reacting polyols, such as diols and triols, e.g., (poly)ethylene glycol ["(poly)" means both cases with and without the prefix "poly"; the same applies below], (poly)propylene glycol, 1,4-butanediol, 1,6-hexanediol, trimethylolpropane, and (poly)glycerin, with unsaturated acids, such as (meth)acrylic acid, maleic acid, and fumaric acid; bisacrylamides, such as N,N-methylenebisacrylamide; di or tri(meth)acrylic acid esters obtained by reacting polyepoxides with (meth)acrylic acid; carbamyl di(meth)acrylates obtained by reacting polyisocyanates, such as tolylene diisocyanate and hexamethylene diisocyanate, with hydroxyethyl (meth)acrylate; compounds having two or more polymerizable unsaturated groups, such as allylated starch, allylated cellulose, diallyl phthalate, N,N',N"-triallyl isocyanurate, and divinylbenzene; polyglycidyl compounds, such as diglycidyl compounds, e.g., (poly)ethylene glycol diglycidyl ether, (poly)propylene glycol diglycidyl ether, and (poly)glycerin diglycidyl ether, and triglycidyl compounds; epihalohydrin compounds, such as epichlorohydrin, epibromohydrin, and α-methylepichlorohydrin; compounds having two or more reactive functional groups, such as isocyanate compounds, e.g., 2,4-tolylene diisocyanate and hexamethylene diisocyanate; and oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are still more preferably used. These internal-crosslinking agents may be used alone or in combinations of two or more.

The amount of the internal-crosslinking agent to be used is preferably 0.000001 to 0.02 mol, more preferably 0.00001 to 0.01 mol, still more preferably 0.00001 to 0.005 mol, and even more preferably 0.00005 to 0.002 mol, per mole of the water-soluble ethylenically unsaturated monomer.

[Hydrocarbon Dispersion Medium]

Examples of the hydrocarbon dispersion medium include $C_{6-8}$ aliphatic hydrocarbons, such as n-hexane, n-heptane, 2-methylhexane, 3-methylhexane, 2,3-dimethylpentane, 3-ethylpentane, and n-octane; alicyclic hydrocarbons, such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons, such as benzene, toluene, and xylene. Among these hydrocarbon dispersion media, n-hexane, n-heptane, and cyclohexane, which are readily industrially available, stable in quality, and inexpensive, are particularly suitably used. These hydrocarbon dispersion media may be used alone or in combinations of two or more. The use of a commercially available mixture of hydrocarbon dispersion media, such as Exxsol Heptane (from Exxon Mobil Corporation; containing 75 to 85% by mass of heptane and its isomeric hydrocarbons), also leads to favorable results.

The amount of the hydrocarbon dispersion medium to be used is preferably 100 to 1500 parts by mass, and more preferably 200 to 1400 parts by mass, per 100 parts by mass of a first-stage water-soluble ethylenically unsaturated monomer, in view of homogeneously dispersing the water-soluble ethylenically unsaturated monomer, and facilitating control of the polymerization temperature. As described below, reversed phase suspension polymerization is performed in one stage (single stage) or two or more multiple stages. The above-mentioned first-stage polymerization refers to the first-stage polymerization reaction in single-stage polymerization or multi-stage polymerization (the same applies below).

[Dispersion Stabilizer]

(Surfactant)

In reversed phase suspension polymerization, a dispersion stabilizer may be used to improve the dispersion stability of the water-soluble ethylenically unsaturated monomer in the hydrocarbon dispersion medium. A surfactant may be used as such a dispersion stabilizer.

Examples of usable surfactants include sucrose fatty acid esters, polyglycerol fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensate polyoxyethylene ethers, polyoxyethylene-polyoxypropylene block copolymers, polyoxyethylene polyoxypropyl alkyl ethers, polyethylene glycol fatty acid esters, alkyl glucosides, N-alkyl glyconamides, polyoxyethylene fatty acid amides, polyoxyethylene alkylamines, polyoxyethylene alkyl ether phosphates, and polyoxyethylene alkyl allyl ether phosphates. Among these surfactants, sorbitan fatty acid esters, polyglycerol fatty acid esters, and sucrose fatty acid esters are particularly preferably used, in view of dispersion stability of the monomer. These surfactants may be used alone or in combinations of two or more.

The amount of the surfactant to be used is preferably 0.1 to 30 parts by mass, and more preferably 0.3 to 20 parts by mass, per 100 parts by mass of the first-stage water-soluble ethylenically unsaturated monomer.

(Polymeric Dispersion Agent)

A polymeric dispersion agent may be used in combination with the above-described surfactant, as the dispersion stabilizer to be used in reversed phase suspension polymerization.

Examples of the polymeric dispersion agent include maleic anhydride modified polyethylene, maleic anhydride modified polypropylene, maleic anhydride modified ethylene-propylene copolymers, maleic anhydride modified EPDM (ethylene-propylene-diene terpolymers), maleic anhydride modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersion agents, maleic anhydride modified polyethylene, maleic anhydride modified polypropylene, maleic anhydride modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used, in view of dispersion stability of the monomer. These polymeric dispersion agents may be used alone or in combinations of two or more.

The amount of the polymeric dispersion agent to be used is preferably 0.1 to 30 parts by mass, and more preferably 0.3 to 20 parts by mass, per 100 parts by mass of the first-stage water-soluble ethylenically unsaturated monomer.

[Other Components]

In the method for producing the water-absorbent polymer particles, other components may be optionally added to the aqueous solution containing the water-soluble ethylenically unsaturated monomer, which is then subjected to reversed phase suspension polymerization. Various additives such as thickeners and chain transfer agents can be added as the other components.

By way of example, a thickener may be added to the aqueous solution containing the water-soluble ethylenically unsaturated monomer, which is then subjected to reversed phase suspension polymerization. By thus adding a thickener to adjust the viscosity of the aqueous solution, the median particle size obtained by reversed phase suspension polymerization can be controlled.

Examples of usable thickeners include hydroxyethylcellulose, hydroxypropylcellulose, methylcellulose, carboxymethylcellulose, polyacrylic acid, (partially) neutralized polyacrylic acid, polyethylene glycol, polyacrylamide, polyethyleneimine, dextrin, sodium alginate, polyvinyl alcohol, polyvinylpyrrolidone, and polyethylene oxide. Assuming that the stirring rate during the polymerization is the same, the higher the viscosity of the aqueous solution containing the water-soluble ethylenically unsaturated monomer, the larger the primary particles and/or secondary particles of the resulting particles tend to be.

[Reversed Phase Suspension Polymerization]

To perform reversed phase suspension polymerization, for example, an aqueous monomer solution containing the water-soluble ethylenically unsaturated monomer is dispersed in a hydrocarbon dispersion medium, in the presence of a dispersion stabilizer. Here, as long as the dispersion stabilizer (a surfactant and a polymeric dispersion agent) is added before the beginning of the polymerization reaction, it may be added either before or after the addition of the aqueous monomer solution.

In particular, in view of readily reducing the amount of the hydrocarbon dispersion medium remaining in the resulting water-absorbent polymer particles, it is preferred to disperse the aqueous monomer solution in the hydrocarbon dispersion medium in which a polymeric dispersion agent is dispersed and then disperse a surfactant therein, followed by polymerization.

Reversed phase suspension polymerization as described above can be performed in one stage or two or more multiple stages. In view of improving productivity, reversed phase suspension polymerization is preferably performed in two or three stages.

Reversed phase suspension polymerization in two or more multiple stages may be performed as follows: the first stage of reversed phase suspension polymerization is performed; subsequently, the water-soluble ethylenically unsaturated monomer is added to and mixed with the reaction mixture obtained by the first-stage polymerization reaction, and the second and subsequent stages of reversed phase suspension polymerization are performed in the same manner as in the first stage. In each of the second and subsequent stages of reversed phase suspension polymerization, it is preferred to add, in addition to the water-soluble ethylenically unsaturated monomer, a radical polymerization initiator within the above-described range of molar ratios of each component relative to the water-soluble ethylenically unsaturated monomer, based on the amount of the water-soluble ethylenically unsaturated monomer added in each of the second and subsequent stages of reversed phase suspension polymerization, and then perform reversed phase suspension polymerization. In the second and subsequent stages of polymerization, an internal-crosslinking agent may also be optionally added to the water-soluble ethylenically unsaturated monomer.

The reaction temperature during the polymerization reaction is preferably 20 to 110°C, and more preferably 40 to 90°C, in view of allowing the polymerization to proceed quickly to reduce the polymerization time for improved economic efficiency, and readily removing the heat of polymerization to perform the reaction smoothly.

<Surface-Crosslinking Step>

Next, the water-absorbent polymer particles of the present invention are obtained by crosslinking the hydrous gel having an internal-crosslinked structure obtained by polymerization of the water-soluble ethylenically unsaturated monomer, by adding a surface-crosslinking agent thereto (surface-crosslinking reaction). The surface-crosslinking reaction is preferably performed in the presence of a surface-crosslinking agent, after the polymerization of the water-soluble ethylenically unsaturated monomer. By thus subjecting the hydrous gel having an internal-crosslinked structure to the surface-crosslinking reaction after the polymerization, it is possible to obtain water-absorbent polymer particles having an increased crosslinking density near the surfaces of the water-absorbent polymer particles to exhibit improvement in various kinds of performance, such as water-absorption capacity under load.

Examples of the surface-crosslinking agent include compounds with two or more reactive functional groups. Examples include polyols, such as ethylene glycol, propylene glycol, 1,4-butanediol, diethylene glycol, triethylene glycol, trimethylolpropane, glycerin, polyoxyethylene glycol, polyoxypropylene glycol, and polyglycerin; polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds, such as epichlorohydrin, epibromohydrin, and $\alpha$-methyl epichlorohydrin; isocyanate compounds, such as 2,4-tolylene diisocyanate and hexamethylene diisocyanate; oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds, such as 1,2-ethylenebisoxazoline; carbonate compounds (e.g., alkylene carbonates), such as ethylene carbonate, propylene carbonate, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxopan-2-one; and hydroxyalkylamide compounds, such as bis[N,N-di($\beta$-hydroxyethyl)]adipamide. Preferred among these surface-crosslinking agents are polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. These surface-crosslinking agents may be used alone or in combinations of two or more.

The amount of the surface-crosslinking agent to be used is preferably 0.00001 to 0.01 mol, more preferably 0.00005 to 0.005 mol, and still more preferably 0.0001 to 0.002 mol, per mole of the total amount of the water-soluble ethylenically unsaturated monomer used for polymerization.

The surface-crosslinking agent may be added as is or as an aqueous solution. Optionally, a solution of the surface-crosslinking agent in a hydrophilic organic solvent may be added. Examples of the hydrophilic organic solvent include lower alcohols, such as methyl alcohol, ethyl alcohol, n-propyl alcohol, and isopropyl alcohol; ketones, such as acetone and methyl ethyl ketone; ethers, such as diethyl ether, dioxane, and tetrahydrofuran; amides, such as N,N-dimethylformamide; and sulfoxides, such as dimethylsulfoxide. These hydrophilic organic solvents may be used alone, in combinations of two or more, or as a solvent mixture with water.

The surface-crosslinking agent may be added at any time after the polymerization reaction of the water-soluble ethylenically unsaturated monomer is substantially completed. The surface-crosslinking agent is preferably added in the presence of 1 to 400 parts by mass of water, more preferably 5 to 200 parts by mass of water, still more preferably 10 to 100 parts by mass of water, even more preferably 20 to 60 parts by mass of water, per 100 parts by mass of the water-soluble ethylenically unsaturated monomer. The amount of water herein refers to the total amount of the water contained in the reaction system and the water optionally used during the addition of the surface-crosslinking agent.

The reaction temperature during the surface-crosslinking reaction is preferably 50 to 250°C, more preferably 60 to 180°C, still more preferably 60 to 140°C, and even more preferably 70 to 120°C. The reaction time of the surface-crosslinking reaction is preferably 1 to 300 minutes, and more preferably 5 to 200 minutes.

<Drying Step>

After reversed phase suspension polymerization is performed as described above, the method may include a drying step of adding external energy, such as heat, to remove the water, the hydrocarbon dispersion medium, and the like by distillation. To remove the water in the hydrous gel after reversed phase suspension polymerization, the system in which the hydrous gel is dispersed in the hydrocarbon dispersion medium is heated to distill the water and the hydrocarbon dispersion medium out of the system by azeotropic distillation. Here, if the distilled hydrocarbon dispersion medium only is returned into the system, continuous azeotropic distillation can be performed. This is preferable in view of preventing deterioration of the resin, because the temperature within the system during drying is maintained at a temperature not higher than the azeotropic temperature with the hydrocarbon dispersion medium. Subsequently, the water and the hydrocarbon dispersion medium are distilled off to obtain water-absorbent polymer particles. By controlling the treatment conditions for the drying step after the polymerization to adjust the amount of water to be removed, various kinds of performance of the resulting water-absorbent polymer particles can be controlled.

In the drying step, the drying treatment by distillation may be performed under atmospheric pressure or reduced pressure. The drying treatment may also be performed in a stream of nitrogen or the like, in view of improving the drying efficiency. When the drying treatment is performed under atmospheric pressure, the drying temperature is preferably 70 to 250°C, more preferably 80 to 180°C, still more preferably 80 to 140°C, and even more preferably 90 to 130°C. When the drying treatment is performed under reduced pressure, the drying temperature is preferably 40 to 160°C, and more preferably 50 to 110°C.

When the surface-crosslinking step with a surface-crosslinking agent is performed after the polymerization of the monomer by reversed phase suspension polymerization, the drying step by distillation is performed as described above, after the completion of the surface-crosslinking step. Alternatively, the surface-crosslinking step and the drying step may be performed simultaneously.

The water-absorbent resin composition of the present invention may contain additives suitable for its purpose. Examples of such additives include inorganic powders, surfactants, oxidizing agents, reducing agents, metal chelating agents, radical chain inhibitors, antioxidants, and anti-bacterial agents. For example, when 0.05 to 5 parts by mass of amorphous silica as an inorganic powder is added to 100 parts by mass of the water-absorbent polymer particles, the flowability of the water-absorbent resin composition can be further improved. The additives are preferably hydrophilic or water-soluble.

In the water-absorbent resin composition of the present invention, the content of the water-absorbent polymer particles (excluding the additives) is preferably 70% by mass or more, more preferably 80% by mass or more, and still more preferably 90% by mass or more.

The water-absorbent resin composition of the present invention can be produced, for example, by mixing the water-absorbent polymer particles, the organic acid, and the porous deodorant in a solid phase.

2. Absorbent Material and Absorbent Article

The water-absorbent resin composition of the present invention constitutes an absorbent material used for hygienic materials, such as sanitary items and disposable diapers, for example, and is suitably used for an absorbent article including the absorbent material. The absorbent article of the present invention is an absorbent article comprising an organic acid, a porous deodorant, and water-absorbent polymer particles, wherein a ratio (X/Y) of an amount X (parts by mass) of the organic acid to an amount Y (parts by mass) of the porous deodorant is 1.0 or more, and wherein a sum (x + y) of a content x (% by mass) of the organic acid and a content y (% by mass) of the porous deodorant based on a total mass of the organic acid, the porous deodorant, and the water-absorbent polymer particles is 0.5% by mass or more. A more detailed structure of the absorbent article of the present invention can be designed as in the above-described water-absorbent resin composition.

Herein, the absorbent material obtained using the water-absorbent resin composition of the present invention contains the particulate water-absorbent resin composition of the present invention. The absorbent material may further contain hydrophilic fibers. Examples of the structure of the absorbent material include a sheet-like structure in which the water-absorbent polymer particles are fixed on a nonwoven fabric or between a plurality of nonwoven fabrics; a mixed dispersion obtained by mixing the particulate water-absorbent resin composition and hydrophilic fibers to give a homogeneous composition; a sandwich structure in which the particulate water-absorbent resin composition is sandwiched between layered hydrophilic fibers; and a structure in which the particulate water-absorbent resin composition and hydrophilic fibers are wrapped in a tissue. The absorbent material may also contain other components, for example, adhesive binders such as thermal bonding synthetic fibers, hot melt adhesives, and adhesive emulsions, for improving the shape retention properties of the absorbent material.

The content of the water-absorbent resin composition in the absorbent material is preferably 5 to 100% by mass, more preferably 10 to 95% by mass, still more preferably 20 to 90% by mass, and even more preferably 30 to 80% by mass.

Examples of the hydrophilic fibers include cellulose fibers, such as cotton-like pulp made from wood, mechanical pulp, chemical pulp, and semi-chemical pulp; artificial cellulose fibers, such as rayon and acetate; and fibers made of synthetic resins, such as hydrophilized polyamides, polyesters, and polyolefins. The hydrophilic fibers typically have an average fiber length of 0.1 to 10 mm. Alternatively, the average fiber length may be 0.5 to 5 mm.

It is possible to obtain the absorbent article of the present invention by holding the absorbent material obtained using the particulate water-absorbent resin composition of the present invention between a liquid-permeable sheet (top sheet) that allows a liquid to pass through and a liquid-impermeable sheet (back sheet) that does not allow a liquid to pass through. The liquid-permeable sheet is positioned on the side that is brought into contact with the body, and the liquid-impermeable sheet is positioned opposite to the side that is brought into contact with the body.

Examples of the liquid-permeable sheet include porous synthetic resin sheets and nonwoven fabrics of the types such as air-through type, spunbond type, chemical bond type, and needle punch type, which are made of fibers of polyethylene, polypropylene, polyester, and the like. Examples of the liquid-impermeable sheet include synthetic resin films made of resins such as polyethylene, polypropylene, and polyvinyl chloride.

3. Additional Matters

The present specification includes at least the inventions as set forth in (1) to (12) below.

(1) A water-absorbent resin composition comprising an organic acid, a porous deodorant, and water-absorbent polymer particles having a structural unit derived from a neutralized salt of an ethylenically unsaturated monomer,

wherein a ratio (X/Y) of an amount X (parts by mass) of the organic acid to an amount Y (parts by mass) of the porous deodorant is 1.0 or more, and
wherein a sum (x + y) of a content x (% by mass) of the organic acid and a content y (% by mass) of the porous deodorant in the total water-absorbent resin composition is 0.50% by mass or more.

(2) The water-absorbent resin composition according to (1) above, wherein the ratio (X/Y) of the amount X (parts by mass) to the amount Y (parts by mass) is 1.0 to 15.0, 1.0 to 12.0, 1.0 to 10.0, 1.0 to 8.0, 1.0 to 6.0, 1.5 to 15.0, 1.5 to 12.0, 1.5 to 10.0, 1.5 to 8.0, 1.5 to 6.0, 2.0 to 15.0, 2.0 to 12.0, 2.0 to 10.0, 2.0 to 8.0, 2.0 to 6.0, 2.5 to 15.0, 2.5 to 12.0, 2.5 to 10.0, 2.5 to 8.0, 2.5 to 6.0, 3.0 to 15.0, 3.0 to 12.0, 3.0 to 10.0, 3.0 to 8.0, or 3.0 to 6.0.

(3) The water-absorbent resin composition according to (1) or (2) above, wherein the sum (x + y) of the content x (% by mass) of the organic acid and the content y (% by mass) of the porous deodorant in the total water-absorbent resin composition is 0.50% by mass to 2.00% by mass, 0.50% by mass to 1.50% by mass, 0.50% by mass to 1.40% by mass, 0.50% by mass to 1.30% by mass, 0.50% by mass to 1.20% by mass, 0.50% by mass to 1.00% by mass, 0.53% by mass to 2.00% by mass, 0.53% by mass to 1.50% by mass, 0.53% by mass to 1.40% by mass, 0.53% by mass to 1.30% by mass, 0.53% by mass to 1.20% by mass, 0.53% by mass to 1.00% by mass, 0.56% by mass to 2.00% by mass, 0.56% by mass to 1.50% by mass, 0.56% by mass to 1.40% by mass, 0.56% by mass to 1.30% by mass, 0.56% by mass to 1.20% by mass, 0.56% by mass to 1.00% by mass, 0.59% by mass to 2.00% by mass, 0.59% by mass to 1.50% by mass, 0.59% by mass to 1.40% by mass, 0.59% by mass to 1.30% by mass, 0.59% by mass to 1.20% by mass, or 0.59% by mass to 1.00% by mass.

(4) The water-absorbent resin composition according to any one of (1) to (3) above, wherein the porous deodorant contains at least one selected from the group consisting of zeolite, activated carbons, silicon dioxide, silicates, titania, alumina, aluminum hydroxide, and magnesium hydroxide.

(5) The water-absorbent resin composition according to any one of (1) to (4) above, wherein the organic acid has a pH of 1.0 to 6.0, 1.0 to 4.0, 1.0 to 3.0, 1.0 to 2.0, 1.5 to 6.0, 1.5 to 4.0, 1.5 to 3.0, or 1.5 to 2.0.

(6) The water-absorbent resin composition according to any one of (1) to (5) above, wherein the organic acid contains at least one selected from the group consisting of tartaric acid, citric acid, malic acid, maleic acid, fumaric acid, succinic acid, acetic acid, and propionic acid.

(7) An absorbent article comprising an organic acid, a porous deodorant, and water-absorbent polymer particles, wherein a ratio (X/Y) of an amount X (parts by mass) of the organic acid to an amount Y (parts by mass) of the porous deodorant is 1.0 or more, and wherein a sum (x + y) of a content x (% by mass) of the organic acid and a content y (% by mass) of the porous deodorant based on a total mass of the organic acid, the porous deodorant, and the water-absorbent polymer particles is 0.5% by mass or more.

(8) The absorbent article according to (7) above, wherein the ratio (X/Y) of the amount X (parts by mass) to the amount Y (parts by mass) is 1.0 to 15.0, 1.0 to 12.0, 1.0 to 10.0, 1.0 to 8.0, 1.0 to 6.0, 1.5 to 15.0, 1.5 to 12.0, 1.5 to 10.0, 1.5 to 8.0, 1.5 to 6.0, 2.0 to 15.0, 2.0 to 12.0, 2.0 to 10.0, 2.0 to 8.0, 2.0 to 6.0, 2.5 to 15.0, 2.5 to 12.0, 2.5 to 10.0, 2.5 to 8.0, 2.5 to 6.0, 3.0 to 15.0, 3.0 to 12.0, 3.0 to 10.0, 3.0 to 8.0, or 3.0 to 6.0.

(9) The absorbent article according to (7) or (8) above, wherein the sum (x + y) of the content x (% by mass) of the organic acid and the content y (% by mass) of the porous deodorant in a total amount of the organic acid, the porous deodorant, and the water-absorbent polymer particle is 0.50% by mass to 2.00% by mass, 0.50% by mass to 1.50% by mass, 0.50% by mass to 1.40% by mass, 0.50% by mass to 1.30% by mass, 0.50% by mass to 1.20% by mass, 0.50% by mass to 1.00% by mass, 0.53% by mass to 2.00% by mass, 0.53% by mass to 1.50% by mass, 0.53% by mass to 1.40% by mass, 0.53% by mass to 1.30% by mass, 0.53% by mass to 1.20% by mass, 0.53% by mass to 1.00% by mass, 0.56% by mass to 2.00% by mass, 0.56% by mass to 1.50% by mass, 0.56% by mass to 1.40% by mass, 0.56% by mass to 1.30% by mass, 0.56% by mass to 1.20% by mass, 0.56% by mass to 1.00% by mass, 0.59% by mass to 2.00% by mass, 0.59% by mass to 1.50% by mass, 0.59% by mass to 1.40% by mass, 0.59% by mass to 1.30% by mass, 0.59% by mass to 1.20% by mass, or 0.59% by mass to 1.00% by mass.

(10) The absorbent article according to any one of (7) to (9) above, wherein the porous deodorant contains at least one selected from the group consisting of zeolite, activated carbons, silicon dioxide, silicates, titania, alumina, aluminum hydroxide, and magnesium hydroxide.

(11) The absorbent article according to any one of (7) to (10) above, wherein the organic acid has a pH of 1.0 to 6.0, 1.0 to 4.0, 1.0 to 3.0, 1.0 to 2.0, 1.5 to 6.0, 1.5 to 4.0, 1.5 to 3.0, or 1.5 to 2.0.

(12) The absorbent article according to any one of (7) to (11) above, wherein the organic acid contains at least one selected from the group consisting of tartaric acid, citric acid, malic acid, maleic acid, fumaric acid, succinic acid, acetic acid, and propionic acid.

Examples

The present invention will be hereinafter described in detail with reference to examples and comparative examples. However, the present invention is not limited to the examples.

The below-described water-absorbent polymer particles, activated carbon serving as a porous deodorant, and water-absorbent resin compositions obtained in the examples and comparative examples were evaluated using the tests as described below. Unless otherwise indicated, the measurement was performed in an environment at a temperature of 25 ± 2°C and a humidity of 50 ± 10%.

(Production Example of Water-Absorbent Polymer Particles)

An 11 cm inner diameter, 2 L volume, cylindrical round-bottomed separable flask was prepared which was equipped with a reflux condenser, a dropping funnel, a nitrogen gas inlet tube, and an agitator with stirring blades having two stages of four inclined paddle blades with a blade diameter of 5 cm. As a hydrocarbon dispersion medium, 293 g of n-heptane was placed in this flask, and 0.736 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C. Separately, in a 300 mL inner volume beaker, 92.0 g (1.03 mol) of an 80.5% by mass aqueous solution of acrylic acid was placed as a water-soluble ethylenically unsaturated monomer, and 147.7 g of a 20.9% by mass aqueous solution of sodium hydroxide was added dropwise, while cooling with ice water, to achieve 75 mol% neutralization. Then, 0.092 g of hydroxyethyl cellulose (HEC AW-15F from Sumitomo Seika Chemicals Co., Ltd.) as a thickener, 0.0736 g (0.272 mmol) of potassium persulfate as a water-soluble radical polymerization agent, and 0.010 g (0.057 mmol) of ethylene glycol diglycidyl ether as an internal-crosslinking agent were added and dissolved to prepare a first-stage aqueous solution. Then, the aqueous solution prepared as above was added to the separable flask and stirred for 10 minutes. Then, a surfactant solution prepared in a 20 mL vial by heating and dissolving 0.736 g of sucrose stearate having an HLB of 3 (Ryoto sugar ester S-370 from Mitsubishi-Kagaku Foods Corporation) as a surfactant in 6.62 g of n-heptane was further added. The system was sufficiently purged with nitrogen while stirring with the agitator at a rotation speed of 550 rpm. Then, the flask was immersed in a water bath at 70°C and heated, and polymerization was performed for 60 minutes to give a first-stage polymerization slurry.

Separately, in another 500 mL inner volume beaker, 128.8 g (1.43 mol) of an 80.5% by mass aqueous solution of acrylic acid was placed as a water-soluble ethylenically unsaturated monomer, and 159.0 g of a 27% by mass aqueous solution of sodium hydroxide was added dropwise, while cooling with ice water, to achieve 75 mol% neutralization. Then, 0.103 g (0.381 mmol) of potassium persulfate as a water-soluble radical polymerization initiator and 0.0117 g (0.067 mmol) of ethylene glycol diglycidyl ether as an internal-crosslinking agent were added and dissolved to prepare a second-stage aqueous solution.

The separable flask system was cooled to 25°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes to give a hydrous gel polymer.

Then, the flask was immersed in an oil bath set at 125°C to remove 260.1 g of water out of the system, while refluxing the n-heptane, by azeotropic distillation of the water and n-heptane. Then, 4.42 g (0.507 mmol) of a 2% by mass aqueous solution of ethylene glycol diglycidyl ether was added to the flask as a surface-crosslinking agent, and the flask was maintained at 83°C for 2 hours.

Then, the n-heptane was evaporated at 125°C to dry the reaction mixture, and the resulting product was subsequently passed through a sieve with a mesh size of 850 μm to give 226.6 g of water-absorbent polymer particles. The water-absorbent polymer particles had a physiological saline retention amount of 42 g/g, a water-absorption rate of 39 seconds, a median particle size of 362 μm, and a physiological saline absorption amount under a load of 4.14 kPa of 20 ml/g.

[Evaluation of Water-Absorbent Polymer Particles]

<Physiological Saline Retention Amount>

2.0 g of the water-absorbent polymer particles were weighed into a cotton bag (Cottonbroad No. 60, 100 mm in width × 200 mm in length), and the cotton bag was placed in a 500 mL beaker. 500 g of a 0.9% by mass aqueous solution of sodium chloride (physiological saline) was poured at once into the cotton bag containing the water-absorbent polymer particles so as to prevent formation of unswollen lumps, then the top of the cotton bag was closed with a rubber band, and the cotton bag was allowed to stand for 30 minutes to cause the water-absorbent polymer particles to swell. After 30 minutes, the cotton bag was dehydrated for 1 minute using a dehydrator (product number: H-122, manufactured by Kokusan Co., Ltd.) set at a centrifugal force of 167 G, and a mass Wd (g) of the cotton bag containing the swollen gel after dehydration was measured. The same operation was performed without adding the water-absorbent polymer particles, and an empty mass We (g) of the cotton bag upon wetting was measured. The physiological saline retention amount was calculated according to the following equation:

$$\text{Physiological saline retention amount (g/g)} = [\text{Wd} - \text{We}]/2.0$$

<Measurement of Pure Water-Absorption Rate by the Vortex Method>

50 ± 0.1 g of pure water adjusted to a temperature of 25 ± 0.2°C in a thermostat was weighed into a 100 ml beaker and stirred with a magnetic stirrer bar (8 mm ϕ × 30 mm, without a ring) to form a vortex at a rotation speed of 600 rpm. 2.0 ± 0.002 g of the water-absorbent polymer particles were added at once into the physiological saline, and the time (seconds) from the addition of the water-absorbent polymer particles to the point at which the vortex on the liquid surface disappeared was measured. The measured time was determined as the water-absorption rate of the water-absorbent polymer particles. This water-absorption rate is also referred to as the Vortex method or vortex time.

<Median Particle Size (Particle Size Distribution)>

50.0 g of the water-absorbent polymer particles were used for median particle size (particle size distribution) measurement. JIS standard sieves having mesh sizes of 850 μm, 500 μm, 425 μm, 300 μm, 250 μm, 180μm, and 150 μm, and a receiving tray were combined in that order from the top. The water-absorbent polymer particles were placed on the top sieve of the combined sieves, and classified by shaking for 20 minutes using a Ro-Tap shaker. After the classification, the particle size distribution was determined by calculating the mass of the water-absorbent polymer particles remaining on each sieve as the mass percentage relative to the total mass. With regard to this particle size distribution, the mass percentage of the water-absorbent polymer particles remaining on each sieve was integrated in descending order of particle size. In this manner, the relationship between the sieve mesh size and the cumulative value of the mass percentage of the water-absorbent polymer particles remaining on each sieve was plotted on logarithmic probability paper. The plots on the probability paper were connected with straight lines, and a particle size equivalent to a 50% by mass cumulative mass percentage was determined as the median particle size.

<Physiological Saline Absorption Amount Under a Load of 4.14 kPa>

Physiological saline absorption amount under a load of 4.14 kPa (water absorption amount under load) was measured using the measurement apparatus as schematically shown in Fig. 1. Two measurements were made for one type of water-absorbent polymer particles, and the average value was obtained. The measurement apparatus includes a burette unit 1, a clamp 3, a conduit 5, a stand 11, a measurement table 13, and a measurement unit 4 placed on the measurement table 13. The burette unit 1 has a graduated burette tube 21, a rubber stopper 23 for sealing the upper opening of the burette tube 21, a cock 22 connected to the lower end of the burette tube 21, an air inlet tube 25 connected to a lower portion of the burette tube 21, and a cock 24. The burette unit 1 is fixed by the clamp 3. The flat measurement table 13 has a through hole 13a with a diameter of 2 mm formed in the central portion thereof, and is supported by the variable-height stand 11. The through hole 13a of the measurement table 13 and the cock 22 of the burette unit 1 are connected through the conduit 5. The conduit 5 has an inner diameter of 6 mm.

The measurement unit 4 includes a cylinder 31 made of plexiglas, a polyamide mesh 32 bonded to one opening of the cylinder 31, and a weight 33 vertically movable within the cylinder 31. The cylinder 31 is placed on the measurement table 13 with the polyamide mesh 32 therebetween. The cylinder 31 has an inner diameter of 20 mm. The polyamide mesh 32 has a mesh size of 75 mm (200 mesh). The weight 33 has a diameter of 19 mm and a mass of 119.6 g and, as described later, can apply a load of 4.14 kPa (0.6 psi) to water-absorbent polymer particles 10a uniformly disposed on the polyamide mesh 32.

First, the cock 22 and the cock 24 of the burette unit 1 were closed, and 0.9% by mass physiological saline adjusted to 25°C was placed in the burette tube 21 through the upper opening of the burette tube 21. Subsequently, the upper opening of the burette tube 21 was sealed with the rubber stopper 23, and then the cock 22 and the cock 24 were opened. The conduit 5 was filled with the 0.9% by mass physiological saline 50 to prevent air bubbles from entering. The height of the measurement table 13 was adjusted so that the height of the surface of the 0.9% by mass physiological saline reached into the through hole 13a was equal to the height of the upper surface of the measurement table 13. After the adjustment, the height of the surface of the 0.9% by mass physiological saline 50 in the burette tube 21 was read on the graduations of the burette tube 21, and this position was determined as a zero point (reading at 0 seconds).

In the measurement unit 4, 0.10 g of the water-absorbent polymer particles 10a were uniformly disposed on the polyamide mesh 32 in the cylinder 31, the weight 33 was disposed on the water-absorbent polymer particles 10a, and the cylinder 31 was installed such that the central portion thereof aligned with the conduit opening at the central portion of the measurement table 13. An amount of reduction Wc (ml) in the physiological saline in the burette tube 21 at 60 minutes after the beginning of absorption of the physiological saline by the water-absorbent polymer particles 10a through the conduit 5 (that is, the amount of the physiological saline absorbed by the water-absorbent polymer particles 10a) was read, and the physiological saline absorption capacity under a load of 4.14 kPa of the water-absorbent polymer particles 10a was calculated using the following equation:

Physiological saline absorption capacity (ml/g) under a load of 4.14 kPa = Wc (ml)/mass (g) of the water-absorbent polymer particles

[Preparation of Organic Acid]

Particulate L-tartaric acid (manufactured by Wego Chemical; median particle size: 206 $\mu$m) was prepared as the organic acid.

[Evaluation of Organic Acid]

<pH Measurement of Organic Acid>

5.0 g of the organic acid and 45.0 g of ion-exchanged water were weighed into a 100 mL plastic beaker. A stirrer bar (8 mm $\phi \times$ 30 mm) was placed in the beaker, and, after 1 hour of stirring at 400 rpm using a magnetic stirrer, the electrode of a portable pH meter (pH/ORP METER D-72, electrode-type 9625, manufactured by HORIBA, Ltd.) was inserted to a position at a depth of 2.5 cm, at a distance of 1 cm away from the inner wall of the beaker. After the insertion, the electrode was allowed to stand for several minutes, and a steady value was determined as the pH of a 10% by mass aqueous solution of the organic acid.

<Median Particle Size of Organic Acid>

10 g of the organic acid was screened using a continuous full-automatic sonic vibration type screening measuring device (Robot Sifter RPS-205, manufactured by Seishin Enterprise Co., Ltd), JIS standard sieves with mesh sizes of 850 μm, 500 μm, 425 μm, 300 μm, 212 μm, 106 μm, 75 μm, and 45 μm, and a receiving tray, under screening conditions at a frequency of 80 Hz, a pulse interval of 1 second, and a classification time of 2 minutes. The mass of the particles remaining on each sieve was calculated as the mass percentage relative to the total mass. The mass percentage of the particles remaining on each sieve was integrated in descending order of particle size. In this manner, the relationship between the sieve mesh size and the cumulative value of the mass percentage of the particles remaining on each sieve was plotted on logarithmic probability paper. The plots on the probability paper were connected with straight lines, and a particle size equivalent to a 50% by mass cumulative mass percentage was determined as the median particle size.

[Preparation of Activated Carbon]

An activated carbon (CARBORAFFIN-6 from Osaka Gas Chemicals Co., Ltd.) having a BET specific surface area of 1345 $m^2$/g, a median particle size of 46 μm, a residue on ignition of 0.4%, a loss on drying of 3.2%, a pH of 4.9, and a crushed shape was prepared.

[Evaluation of Activated Carbon]

<Median Particle Size (Laser Diffraction) of Activated Carbon>

The median particle size (D50 (median size), volume-based) of the activated carbon used was measured using a laser diffraction particle size distribution analyzer (SALD-2300, manufactured by Shimadzu Corporation).

<BET Specific Surface Area of Activated Carbon>

0.1 g of the activated carbon to be measured was dried under degassing conditions of evacuation with heating at 60°C for 24 hours, using a pretreatment apparatus (BELPREP VAC II; MicrotracBel Corporation). Then, an adsorption isotherm was measured at a temperature of 77 K using a specific surface area analyzer (BELSORP MINI II from MicrotracBel Corporation), by the method using nitrogen gas as the adsorption gas, and the specific surface area was determined from a multi-point BET plot as the BET specific surface area of the activated carbon.

[Production of Water-Absorbent Resin Compositions]

<Example 1>

To 100 parts by mass of the water-absorbent polymer particles obtained in the production example, 0.5 parts by mass of L-tartaric acid mentioned above as the organic acid and 0.1 parts by mass of the activated carbon mentioned above as the porous deodorant were added, and the mixture was mixed for 30 minutes using a cross-rotary mixer manufactured by Meiwa Kogyo Co., Ltd., at a rotation speed of 50 rpm and a revolution speed of 50 rpm, to give a water-absorbent resin composition.

<Example 2>

A water-absorbent resin composition was obtained as in Example 1, except that the amount of the activated carbon added was 0.30 parts by mass.

<Example 3>

A water-absorbent resin composition was obtained as in Example 1, except that the amount of L-tartaric acid added was 1.0 parts by mass.

<Example 4>

A water-absorbent resin composition was obtained as in Example 1, except that the amount of L-tartaric acid added was 1.00 part by mass, and the amount of the activated carbon added was 0.30 parts by mass.

<Example 5>

A water-absorbent resin composition was obtained as in Example 1, except that the amount of L-tartaric acid added was 0.75 parts by mass, and the amount of the activated carbon added was 0.20 parts by mass.

<Comparative Example 1>

A water-absorbent resin composition was obtained as in Example 1, except that the amount of L-tartaric acid added was 0.25 parts by mass, and the amount of the activated carbon added was 0.05 parts by mass.

<Comparative Example 2>

A water-absorbent resin composition was obtained as in Example 1, except that the amount of L-tartaric acid added was 1.00 part by mass, and the amount of the activated carbon added was 0.05 parts by mass.

<Comparative Example 3>

A water-absorbent resin composition was obtained as in Example 1, except that the amount of L-tartaric acid added was 0.25 parts by mass, and the amount of the activated carbon added was 0.30 parts by mass.

<Comparative Example 4>

A water-absorbent resin composition was obtained as in Example 1, except that the amount of L-tartaric acid added was 0.25 parts by mass, and the activated carbon was not added.

<Comparative Example 5>

A water-absorbent resin composition was obtained as in Example 1, except that the amount of L-tartaric acid added was 0.50 parts by mass, and the activated carbon was not added.

<Comparative Example 6>

A water-absorbent resin composition was obtained as in Example 1, except that the amount of L-tartaric acid added was 0.75 parts by mass, and the activated carbon was not added.

<Comparative Example 7>

A water-absorbent resin composition was obtained as in Example 1, except that the amount of L-tartaric acid added was 1.00 part by mass, and the activated carbon was not added.

<Comparative Example 8>

A water-absorbent resin composition was obtained as in Example 1, except that L-tartaric acid was not added, and the amount of the activated carbon added was 0.05 parts by mass.

<Comparative Example 9>

A water-absorbent resin composition was obtained as in Example 1, except that L-tartaric acid was not added, and the amount of the activated carbon added was 0.10 parts by mass.

<Comparative Example 10>

A water-absorbent resin composition was obtained as in Example 1, except that L-tartaric acid was not added, and the amount of the activated carbon added was 0.20 parts by mass.

<Comparative Example 11>

A water-absorbent resin composition was obtained as in Example 1, except that L-tartaric acid was not added, and the amount of the activated carbon added was 0.30 parts by mass.

<Reference Example 1>

The water-absorbent polymer particles obtained in the production example were used as Reference Example 1.

[Evaluation of Water-Absorbent Resin Compositions]

<Ammonia Generation Prevention Test>

Artificial urine was prepared by dissolving 25.0 g of urea, 9.0 g of sodium chloride, 0.6 g of magnesium sulfate heptahydrate, 0.7 g of calcium lactate, 4.0 g of potassium sulfate, 2.5 g of ammonium sulfate, and 0.1 g of L-cystine in 958.1 g of distilled water. A urease solution was also prepared by diluting urease (1000 U/mL of a 50% glycerin solution from Jack bean (*Canavalia ensiformis*), manufactured by MERCK) with distilled water to 2 U/mL. In a sterile petri dish (diameter 88 mm, height 17 mm), 1.00 g of the water-absorbent resin composition or the water-absorbent polymer particles were placed, and a test liquid (prepared by mixing 45.0 mL of the artificial urine and 1.0 mL of the urease solution) was added to cause the sample to swell. After the addition of the test liquid, the sample was sealed in a 2 L sampling bag made of polyester (PAAAK2, manufactured by GL Sciences Inc.), the air in the bag was removed, and instead, 900 mL of dry air was added into the bag. The bag was subsequently stored at 35°C, and, after 24 hours, the ammonia concentration was measured using gas detecting tubes (Ammonia 3L, 3La, 3M, manufactured by Gastec Corporation). Table 1 shows the deodorization ratio determined based on this measured value and the following equation 1:

Deodorization ratio (%) = [(ammonia concentration in Reference Example 1 - ammonia concentration in the example or comparative example)/ammonia concentration in Reference Example 1] × 100          Equation 1:

<Degree of Dusting Test>

A 500 mL capacity glass suction bottle was prepared. A SUS hopper (88 mm in top inner diameter × 18 mm in foot inner diameter) was placed so that the height from the bottom of the suction bottle to the discharge port of the hopper was 180 mm, and the suction port of the suction bottle was connected to a dust meter (digital indicator model LD-5R, manufactured by Sibata Scientific Technology Ltd.) with a glass tube (7.7 mm in inner diameter × 300 mm in length). As a sample, 3.0 g ± 0.1 g of the water-absorbent resin composition was charged into the hopper, the damper of the hopper was pulled out and the start button of the dust meter was pressed simultaneously, and counters after 1 minute (counters (A) for the sample) were recorded. Before the sample was measured, a blank test was performed to determine counters (B) in the blank test, and the degree of dusting was calculated based on the following equation:

$$\text{Degree of dusting (cpm)} = A - B$$

where A represents the counters (cpm) for the sample, and B represents the counters (cpm) in the blank test. For one type of sample, the degree of dusting was measured three times as described above, and the average value was adopted as the degree of dusting of the sample. The results are shown in Table 2.

[Table 1]

| | Water-Absorbent Resin Composition | | | | | | | | | Ammonia Concentration [ppm] | Deodorization Ratio [%] |
|---|---|---|---|---|---|---|---|---|---|---|---|
| | Water-Absorbent Polymer Particles [Parts by Mass] | Organic Acid | Amount X [Parts by Mass] of Organic Acid | Content x [% by Mass] of Organic Acid | Porous Deodorant | Amount Y [Parts by Mass] of Porous Deodorant | Content y [% by Mass] of Porous Deodorant | X/Y | x+y [% by Mass] | | |
| Ex.1 | 100 | L-Tartaric Acid | 0.50 | 0.50 | Activated Carbon | 0.10 | 0.10 | 5.0 | 0.60 | 140 | 53 |
| Ex.2 | 100 | L-Tartaric Acid | 0.50 | 0.50 | Activated Carbon | 0.30 | 0.30 | 1.7 | 0.79 | 84 | 72 |
| Ex.3 | 100 | L-Tartaric Acid | 1.00 | 0.99 | Activated Carbon | 0.10 | 0.10 | 10 | 1.09 | 90 | 70 |
| Ex.4 | 100 | L-Tartaric Acid | 1.00 | 0.99 | Activated Carbon | 0.30 | 0.30 | 3.3 | 1.28 | 56 | 81 |
| Ex.5 | 100 | L-Tartaric Acid | 0.75 | 0.74 | Activated Carbon | 0.20 | 0.20 | 3.8 | 0.94 | 78 | 74 |
| Comp. Ex.1 | 100 | L-Tartaric Acid | 0.25 | 0.25 | Activated Carbon | 0.05 | 0.05 | 5.0 | 0.30 | 230 | 23 |
| Comp. Ex.2 | 100 | L-Tartaric Acid | 1.00 | 0.99 | Activated Carbon | 0.05 | 0.05 | 20 | 1.04 | 150 | 50 |
| Comp. Ex.3 | 100 | L-Tartaric Acid | 0.25 | 0.25 | Activated Carbon | 0.3 | 0.30 | 0.8 | 0.55 | 140 | 53 |
| Comp. Ex.4 | 100 | L-Tartaric Acid | 0.25 | 0.25 | - | 0 | 0 | - | 0.25 | 280 | 7 |
| Comp. Ex.5 | 100 | L-Tartaric Acid | 0.50 | 0.50 | - | 0 | 0 | - | 0.50 | 250 | 17 |
| Comp. Ex.6 | 100 | L-Tartaric Acid | 0.75 | 0.74 | - | 0 | 0 | - | 0.74 | 220 | 27 |
| Comp. Ex.7 | 100 | L-Tartaric Acid | 1.00 | 0.99 | - | 0 | 0 | - | 0.99 | 200 | 33 |

| | Water-Absorbent Resin Composition | | | | | | | | | Ammonia Concentration [ppm] | Deodorization Ratio [%] |
|---|---|---|---|---|---|---|---|---|---|---|---|
| | Water-Absorbent Polymer Particles [Parts by Mass] | Organic Acid | Amount X [Parts by Mass] of Organic Acid | Content **x** [% by Mass] of Organic Acid | Porous Deodorant | Amount Y [Parts by Mass] of Porous Deodorant | Content y [% by Mass] of Porous Deodorant | X/Y | x+y [% by Mass] | | |
| Comp. Ex.8 | 100 | - | 0 | 0 | Activated Carbon | 0.05 | 0.05 | 0.0 | 0.05 | 240 | 20 |
| Comp. Ex.9 | 100 | - | 0 | 0 | Activated Carbon | 0.10 | 0.10 | 0.0 | 0.10 | 200 | 33 |
| Comp. Ex.10 | 100 | - | 0 | 0 | Activated Carbon | 0.20 | 0.20 | 0.0 | 0.20 | 170 | 43 |
| Comp. Ex.11 | 100 | - | 0 | 0 | Activated Carbon | 0.30 | 0.30 | 0.0 | 0.30 | 160 | 47 |
| Ref. Ex.1 | 100 | - | 0 | 0 | - | 0 | 0 | - | 0 | 300 | - |

[Table 2]

| | | Water-Absorbent Resin Composition | | |
| --- | --- | --- | --- | --- |
| | | Synergistic Effect of Deodorization Ratio (Reference 1.00) | Degree of Dusting | Pure Water-Absorption Rate (vortex) [sec.] |
| | Ex.1 | 1.06 | 458 | 18 |
| | Ex.2 | 1.13 | 1022 | 18 |
| | Ex.3 | 1.06 | 488 | 21 |
| | Ex.4 | 1.01 | 1069 | 20 |
| | Ex.5 | 1.06 | 792 | 19 |
| | Comp. Ex.1 | 0.85 | - | - |
| | Comp. Ex.2 | 0.94 | - | - |
| | Comp. Ex.3 | 0.98 | - | - |
| | Ref. Ex.1 | - | - | 16 |

As shown in Table 1, for example, in Comparative Example 11 in which the content y of the porous deodorant in the water-absorbent resin composition was 0.30% by mass (the organic acid was not contained), the deodorization ratio was 47%, and in Comparative Example 5 in which the content x of the organic acid in the water-absorbent resin composition was 0.50% by mass (the porous deodorant was not contained), the deodorization ratio was 17%. On the other hand, in Example 2 in which the content y of the porous deodorant was 0.30% by mass and the content y of the organic acid was 0.50% by mass in the water-absorbent resin composition, the deodorization ratio was as high as 72%, which shows that a synergistic effect between the organic acid and the porous deodorant was exhibited, which was unexpected from the results of Comparative Examples 5 and 11 (even the sum of the deodorization ratios in Comparative Examples 5 and 1 was 64%). The synergistic effect on the deodorization ratio of each water-absorbent resin composition shown in Table 2 is obtained by dividing the deodorization ratio of the example by the sum of the deodorization ratios of the comparative examples in which the respective deodorants were used alone. For example, the synergistic effect of Example 2 is the value calculated by: deodorization ratio (72) of Example 2/(deodorization ratio (47) of Comparative Example 11 + deodorization ratio (17) of Comparative Example 5). When the value exceeds the reference value 1.00, it can be said that a synergistic effect is exhibited.

In addition, typically, the addition of an additive to the water-absorbent polymer particles slows down the water-absorption rate; however, the results shown in Table 2 show that the water-absorbent resin compositions of Examples 1 to 5 maintained a water-absorption rate close to that of Reference Example 1 (containing no deodorant). The results shown in Table 2 also show that among the water-absorbent resin compositions of Examples 1 to 5, Examples 1, 3, and 5, particularly Example 1, are excellent in the degree of dusting.

Reference Signs List

1: burette unit
3: clamp
4: measurement unit
5: conduit
10a: water-absorbent polymer particles
11: stand
13: measurement table
13a: through hole
21: burette tube
22: cock
23: rubber stopper
24: cock
25: air inlet tube
31: cylinder
32: polyamide mesh

33: weight
50: saline

## Claims

1. A water-absorbent resin composition comprising an organic acid, a porous deodorant, and water-absorbent polymer particles having a structural unit derived from a neutralized salt of an ethylenically unsaturated monomer,

   wherein a ratio (X/Y) of an amount X (parts by mass) of the organic acid to an amount Y (parts by mass) of the porous deodorant is 1.0 or more, and
   wherein a sum (x + y) of a content x (% by mass) of the organic acid and a content y (% by mass) of the porous deodorant in the total water-absorbent resin composition is 0.50% by mass or more.

2. The water-absorbent resin composition according to claim 1, wherein the ratio (X/Y) of the amount X (parts by mass) to the amount Y (parts by mass) is 12.0 or less.

3. The water-absorbent resin composition according to claim 1 or 2, wherein the sum (x + y) of the content x (% by mass) of the organic acid and the content y (% by mass) of the porous deodorant is 1.50% by mass or less.

4. An absorbent article comprising an organic acid, a porous deodorant, and water-absorbent polymer particles,

   wherein a ratio (X/Y) of an amount X (parts by mass) of the organic acid to an amount Y (parts by mass) of the porous deodorant is 1.0 or more, and
   wherein a sum (x + y) of a content x (% by mass) of the organic acid and a content y (% by mass) of the porous deodorant based on a total mass of the organic acid, the porous deodorant, and the water-absorbent polymer particles is 0.5% by mass or more.

[FIG. 1]

## INTERNATIONAL SEARCH REPORT

| International application No. |
|---|
| **PCT/JP2024/003049** |

| A. | CLASSIFICATION OF SUBJECT MATTER |
|---|---|

*B01J 20/26*(2006.01)i; *A61L 9/014*(2006.01)i; *C08K 5/09*(2006.01)i; *C08K 7/22*(2006.01)i; *C08L 101/14*(2006.01)i
FI:   B01J20/26 D; A61L9/014; C08K5/09; C08K7/22; C08L101/14

According to International Patent Classification (IPC) or to both national classification and IPC

| B. | FIELDS SEARCHED |
|---|---|

Minimum documentation searched (classification system followed by classification symbols)

B01J20/10; B01J20/20; B01J20/26; A61L9/014; C08K5/09; C08K7/22; C08L101/14

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2011/162244 A1 (ADACHI, Kanichi) 29 December 2011 (2011-12-29) claims 1-3, paragraphs [0047], [0058], [0061], [0067], [0073], examples 1, 11 | 1-4 |
| A | JP 2023-502995 A (STICHTING VOOR DE TECHNISCHE WETENSCHAPPEN) 26 January 2023 (2023-01-26) entire text, all drawings | 1-4 |

☐ Further documents are listed in the continuation of Box C.      ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **22 March 2024** | **02 April 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/003049**

| Patent document cited in search report | | | Publication date (day/month/year) | Patent family member(s) | | | Publication date (day/month/year) |
|---|---|---|---|---|---|---|---|
| WO | 2011/162244 | A1 | 29 December 2011 | CN | 102947231 | A | |
| | | | | KR 10-2013-0086543 | | A | |
| JP | 2023-502995 | A | 26 January 2023 | US | 2022/0401889 | A1 | |
| | | | | entire text, all drawings | | | |
| | | | | EP | 4061519 | A1 | |
| | | | | CA | 3158273 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001323155 A